# EUROPÄISCHE PATENTANMELDUNG

(11) **EP 1 950 225 A1**
(43) Veröffentlichungstag der Anmeldung: **30.07.2008**
(21) Anmeldenummer: 07101200.9
(22) Anmeldetag: 25.01.2007
(51) Int. Cl.: C07K 16/06, C12P 21/00

(54) **Verfahren zur Steigerung von Proteinausbeuten**

(71) Anmelder: Octapharma AG, 8853 Lachen (CH)
(72) Erfinder: Maguerre, Wolfgang, 69120 Heidelberg (DE)
(74) Vertreter: Meyers, Hans-Wilhelm

(57) **Zusammenfassung**

Verfahren zur Steigerung der Ausbeute von Proteinen, insbesondere Plasmaproteinen aus die Proteine enthaltenden Quellen, wobei die Proteine enthaltenden Quellen bei Temperaturen ≤ -70 C eingefroren werden und die Proteine aus einer aufgetauten eingefrorenen Quelle in an sich bekannter Weise weiter aufgearbeitet werden.

## Beschreibung

Gegenstand der vorliegenden Erfindung ist ein Verfahren zur Steigerung der Ausbeute von Proteinen, insbesondere Plasmaproteinen aus die Proteine enthaltenden Quellen.

Proteine, insbesondere Plasmaproteine enthaltende Quellen sind ein wertvoller Rohstoff für die Gewinnung lebenswichtiger Faktoren, die Patienten mit angeborenen oder erworbenen Mangelzuständen in Form von Konzentraten verabreicht werden. Entsprechende Konzentrate dieser Faktoren oder Kombinationen dieser Proteine werden zur Therapie und Prophylaxe verschiedener Indikationen angewendet. Beispielhaft seien hier Konzentrate des Gerinnungsfaktors VIII und Faktors IX genannt, die zur Behandlung der Hämophilie A bzw. B eingesetzt werden, von Willebrand Faktor enthaltende Fraktionen zur Behandlung der sogenannten von Willebrand Krankheit oder Fibrinogen- und Prothrombin-Komplex-Konzentrate für den angeborenen und erworbenen Mangel von einzelnen und kombinierten Defizienzen der entsprechenden Faktoren. Inhibitoren wie Alpha-1-Antitrypsin, Antithrombin III oder C1-esterase Inhibitor stellen in Form von Konzentraten lebensrettende Medikamente dar, da sie proteolytische Reaktionen limitieren und far die Hämostase wichtige Systeme regulieren. Darüber hinaus können sie auch entzündungshemmende und weitere regulatorische Funktionen vermitteln. Albumin erfüllt ebenfalls wichtige Funktionen bei der Aufrechterhaltung des Blut-Plasma-Systems und stellt durch seine Transportfunktion von physiologischen und der Bindung von toxischen Substanzen einen essentiellen Bestandteil der Regulation dar. Immunglobulin-Konzentrate werden über die Substitution von erblich oder erworbenen Mangelzuständen auch zur Prophylaxe und Behandlung verschiedener entzündlicher und dysregulierter immunologischer Reaktionen verabreicht. Dies wird in der Regel durch intravenöse, subkutane oder intramuskuläre Administration erzielt.

Über diese beispielhaft erwähnten Plasmabestandteile hinaus, im Wesentlichen Konzentrate unterschiedlicher Reinheit, werden andere einzelne Komponenten und deren Kombinationen bereits eingesetzt oder werden noch untersucht. Darunter befinden sich Konzentrate einzelner oder kombinierter Faktoren, wie zur Blutgerinnung verwendete Proteine, in nicht-aktivierter oder aktivierter Form, Proteine zur Förderung der Wundheilung, Immunglobulin-Konzentrate definierter Spezifität, Klassen (IgG, IgA, IgM, IgE) bzw. Subklassen oder daraus hergestellter Modifikationen etc.

Plasma stellt eine Quelle verschiedener wertvoller und lebensrettender Bestanteile dar. Die Fraktionierung des Plasmas und Gewinnung einzelner Konzentrate oder Kombinationen von im Wesentlichen Proteinen wird durch dem Fachmann bekannte Verfahren durchgeführt, wie dem sogenannten Verfahren nach Cohn oder Kistler-Nitschmann, die durch Variation und Optimierung von Temperatur, pH-Wert und Ethanolkonzentration zur Anreicherung von bestimmten Plasmabestandteilen führt, wenn eine entsprechende Separation mittels Fällung und Abtrennung durch Filtration, Zentrifugation oder andere geeignete Maßnahmen durchgeführt wird. Zweck dieser Präzipitation unter definierten Bedingungen ist die Anreicherung eines oder mehrer Plasmabestandteile, wobei eine vollständige Separation und Reinigung von anderen Plasmabestandteilen allein durch einen solchen Schritt häufig nicht in vollem Umfang erreicht wird. Entsprechend werden vor allem die Zielkomponenten, wie Immunglobuline, durch Zugabe geeigneter Lösungen wieder in Lösung gebracht. um gegebenenfalls weitere Verfahrensschritte anzuschließen, die zum endgültigen Konzentrat führen. Diese Schritte bestehen zumeist aus weiteren selektiven Fällungen, Filtrationen und/oder chromatographischen Verfahren. Bestandteil des Herstellungsverfahrens sind Schritte zur Inaktivierung und Abtrennung potentiell infektiöser Bestandteile, wie Viren und infektiöse Prionen. Dem Fachmann sind beispielsweise Inaktivierungen durch das sogenannte Solvens-Detergenz-Verfahren (EP-A-131 740), Pasteurisierung, Behandlung mit inaktivierenden Substanzen und anschliessender Abtrennung, Inkubation bei saurem pH Wert, UVC-Bestrahlung, Nanofiltration oder andere selektive Verfahren bekannt.

Die Präparation der Konzentrate beginnt zumeist mit dem Vereinigen, dem 'Poolen' von Plasma-Einzelspenden. Die gefrorenen Einzelspenden, in der Regel Hunderte bis viele Tausende pro Batch, werden vereint und unter definierten Bedingungen aufgetaut, so dass das sogenannte Cryo-Präzipitat erhalten wird, das die Gerinnungsfaktoren FVIII und von Willebrand Faktor in angereicherter Form enthält, jedoch auch als Quelle für die Gewinnung von Fibrinogen und weiterer Plasmaproteine wie Flbronektin dienen kann.

Nach Abtrennung des Cryo-Präzipitates verbleibt das sogenannte cryo-arme Plasma zumeist als Ausgangsmaterial für die Präparation der anderen Konzentrate. Die o.g. Verfahren zur Separation von Hauptfraktionen nach Cohn (Cryopräzipitat, Fraktion I, Fraktion II+III, Fraktion I+II+III, Fraktion II, Fraktion I+III, Fraktion III, Fraktion IV, Fraktion V) und Kistler-Nitschmann (Cryopräzipitat, Fraktionen I, IV, Präzipitate A, B, C, D, G(G)) reichern die Zielkomponenten, wie Fibrinogen, Faktor XIII, Immunglobuline, Albumin, Alpha-1Antitrypsin u.a., in bestimmten Ausführungen auch andere Inhibitoren wie Antithrombin III. in den verschiedenen Präzipitaten an, die wie o.g. gewonnen werden können.

Alternativ oder zusätzlich zu den Präzipitationen werden Adsorptionen mit dem Fachmann bekannten Verfahren und Matrices durchgeführt, darunter zumeist sogenannte Chromatographie-Gele oder spezielle Filter, die unterschiedliche Eigenschaften aufweisen können, um den spezifischen Charakteristika der Zielproteine gerecht zu werden und eine möglichst effektive Gewinnung zu ermöglichen. Beispielsweise werden typischerweise Gerinnungsfaktor IX oder Prothrombinkomplex Faktoren (FII, FVII, FIX, FX und zusätzlich die Proteine C, S und Z) ohne vorherige Fällung mit Hilfe von Gelen, die Eigenschaften eines Anionenaustauschers haben, aus dem cryoarmen Plasma gewonnen. Antithrombin III kann durch Adsorption an immobilisiertes Heparin effektiv angereichert werden, häufig nach vorheriger Präzipitation anderer Plasmabestandteile. Alternative Verfahren gewinnen jedoch Antithrombin III auch aus speziellen Präzipitaten.

Aus dem Cryo-Präzipitat kann Fibrinogen, FVIII, VWF oder die Kombination Faktor VIII/VWF gewonnen werden, indem die im gelösten Cryo-Präzipitat befindlichen Bestandteile weiter gereinigt werden, prinzipiell mit oben genannten Verfahrensschritten. Cryo-Präzipitat wird jedoch auch als solches in gelöster Form therapeutisch verwendet, wobei auch hier von großer Wichtigkeit eine optimale Ausbeute der benötigten Faktoren und deren Unversehrtheit ist.

Wie ausgeführt stellt z.B. Blutplasma die Quelle vieler Medikamente, meist in Form von Konzentraten speziell angereicherter Faktoren bzw. Proteine, dar. Die effektive Nutzung dieses wertvollen Ausgangsmateriales erfordert entsprechend die Separation in die verschiedenen Hauptfraktionen und deren weitere Verarbeitung zu den entsprechenden Endprodukten. Durch die Vielfalt der o.g. Faktoren und möglichen Produkte aus einem Plasmapool ist die genaue Planung und häufig parallele Durchführung unterschiedlicher Verfahren erforderlich. Entsprechend ist ein hohes Maß an Aufwand, Organisation und Infrastruktur notwendig. Wenn eine solche parallele Weiterverarbeitung aller anfallenden Intermediate zu den jeweiligen Endprodukten nicht möglich ist, müssen verschiedene Herstellungs-Intermediate adäquat gelagert werden. Nicht selten werden äquivalente Intermediate verschiedener Batches auch vereinigt, um die sich daran anschliessenden Verfahrenschritte und die Effektivität zu optimieren.

Da es sich in vielen Fällen um Intermediate handelt, die als Präzipitat oder in Lösung nur für bestimmte Zeiten gelagert werden können, ohne dass das Zielprotein Schaden nimmt, werden die Präzipitate oder flüssigen Intermediate eingefroren und entsprechend gelagert. Abhängig vom Ausgangsvolumen des Plasma-Pools handelt es sich dabei nicht selten um sehr große Volumina bzw. Präzipitatmassen. Entsprechend werden u.a. aus technischen Gründen die Lösungen häufig unter Bedingungen eingefroren und gelagert, die selten unter -30° C liegen.

Das sogenannte "Schockfrieren" von Plasma in flüssigem Stickstoff wurde als vorteilhaft beschrieben, da es besonders die Gewinnung des Gerinnungsfaktors VIII gegenüber herkömmlichen Methoden optimieren sollte. Eine effektivere Ausbeute an FVIII wurde nach Rekonstitution des gewonnenen Cryo-Präzipitates erzielt. Das Einfrieren von Intermediaten, wie dem Cryo-Präzipitat selbst, oder anderen Intermediaten (selbst aus schockgefrorenem Plasma) im Rahmen der Produktherstellung bei sehr tiefen Temperaturen wurde dagegen nicht offenbart.

Ohne durch theoretische Überlegungen festgelegt zu sein, kann ein möglicher Grund dafür darin gesehen werden, dass die 'Erwärmung' von Lösungen und Präzipitaten, die bei sehr niedrigen Temperaturen eingefroren wurden, für die Lagerung in Temperaturbereiche unter -70°C und beim Auftauvorgang als eher nicht vorteilhaft erachtet wird, da eine Umstrukturierung der kristallinen Formen des gefrorenen Gutes bei solchen Temperaturübergängen erfolgen kann und sich damit eher nachteilig auf die Nativität und Aktivitäten von Produktkomponente(n) auswirken könnten, und damit auch mindernd auf die Produktausbeuten.

Die Optimierung bestehender Herstellprozesse im Sinne einer Ausbeutesteigerung der Zielproteine und Beibehaltung der hohen Qualität ist eine Aufgabe, deren Lösung sich die vorliegende Erfindung zum Ziel gesetzt hat.

Gelöst wird das der Erfindung zu Grunde liegende Problem durch ein Verfahren zur Steigerung der Ausbeute von Proteinen, insbesondere Plasmaproteinen aus die Proteine, insbesondere Plasmaproteine enthaltenden Quellen, wobei die Proteine enthaltenden Quellen bei Temperaturen ≤ -70° C eingefroren werden und die Proteine aus einer aufgetauten eingefrorenen Quelle in an sich bekannter Weise weiter aufgearbeitet werden.

Überraschenderweise wurde gefunden, dass das Einfrieren von proteinhaltigen Fraktionen wie Proteinpräzipitaten und proteinhaltigen Lösungen bei Temperaturen ≤ -70° C zu einer Ausbeutesteigerung in den aufgetauten Intermediaten und den resultierenden Endprodukten führt.

In einer Ausführungsform des erfindungsgemäßen Verfahrens werden die Proteine z.B. Plasmaproteine enthaltenden Quellen mit flüssigem Stickstoff eingefroren.

Erfindungsgemäß werden als Plasmaproteine solche aus der Gruppe bestehend aus Immunglobulinen aller Klassen und Subtypen, Gerinnungsfaktoren, andere in Gerinnung oder Fibrinolyse involvierte Proteine, Albumin und Wundverschluss oder Wundheilung fördernde Substanzen sowie Proteine mit Transportfunktion ausgewählt.

Dabei kommen als Immunglobuline IgG, IgM, IgA, IgE und deren Subklassen in Betracht.

Erfindungsgemäß kann die Ausbeute der Gerinnungsfaktoren Faktoren II, V, VII, VIII, IX, X, XI, XII, XIII, Fibrinogen und von Willebrand Faktor erhöht werden.

Die anderen in Gerinnung oder Fibrinolyse involvierten Proteine sind erfindungsgemäß insbesondere Plasminogen, Faktor VII aktivierende Protease, Proteaseinhibitoren, wie Alpha-1-Antitrypsin, Antithrombin III oder C 1-Esteraseinhibitor und Alpha-2-Antiplasmin.

Die den Wundverschluss oder die Wundheilung fördernden Substanzen sind insbesondere Fibronektin, Wachstumsfaktoren wie HGF, FGF oder PDGF.

Als Proteine mit Transportfunktion, die erfindungsgemäß mit verbesserter Ausbeute gewonnen werden können sind insbesondere Transferrin, Faktoren des Komplementsystemes oder Histidin-reiches Glykoprotein zu nennen.

Für das erfindungsgemäße Verfahren werden in einer Ausführungsform als die Plasmaproteine enthaltenden Quellen Cryopräzipitat, Cohn Fraktionen I, II, III, I+III, II+III, I+II+III, IV, V sowie Kombinationen davon oder Kistler-Nitschmann Fraktionen I, IV, Präzipitaten A, B, C, D, G(G) sowie deren Kombinationen und Modifikationen ausgewählt.

Die im erfindungsgemäßen Verfahren einsetzbaren Proteine, insbesondere Plasmaproteine enthaltenden Quellen können beispielsweise aus Protein-Präzipitaten erhältlich sein, die die herzustellenden Plasmaproteine zu weniger als 50%, insbesondere 30% bzw. 10% denaturieren.

Dabei können die Protein-Präzipitate durch Zugabe von Polyethylenglykol zu den die Proteine, insbesondere Plasmaproteine enthaltenden Quellen und/oder Aussalzen von Proteinen aus den die Proteine enthaltenden Quellen erhältlich sein.

In einer weiteren Ausführungsform des erfindungsgemäßen Verfahrens kann eine Lagerung der bei Temperaturen ≤ -70°C eingefrorenen Proteine, insbesondere Plasmaproteine enthaltenden Quellen bei Temperaturen ≤ - 18°C, insbesondere bei ≤ -70°C, erfolgen.

Das erfindungsgemäße Verfahren hat sich insbesondere für die Herstellung von Immunglobulinen bewährt, wobei die zur Gewinnung der Immunglobuline eingesetzten Ausgangsmaterialien bei Temperaturen ≤ -70°C und auch deren Lagerung bei Temperaturen ≤ -70°C erfolgten. Auch Cryopräzipitat wird vorteilhafterweise bei Temperaturen ≤ -70°C eingefroren bzw. gelagert.

Typischerweise werden Plasmaproteine enthaltende Lösungen, Pasten Intermediate aus der Cohn-Fraktionierung oder Kistler-Nitschmann Fraktionierung stammenden Fraktionen eingefroren. Auch proteinhaltige Fraktionen aus rekombinant und transgen hergestellten Präzipitaten und Lösungen können eingefroren und gelagert werden. Die Lagerung kann dabei für eine Zeit von mindestens 12 Stunden erfolgen.

Nach dem erfindungsgemässen Verfahren können die Proteine, insbesondere Plasmaproteine enthaltenden Quellen in Form von Proteinpräzipitaten vorliegen, die in unterschiedlicher Weise gewonnen werden. Darunter sind dem Fachmann vertraute Verfahren zur Präzipitation nach Cohn und Kistler-Nitschmann (KN) bekannt, jedoch auch durch Polyethylenfällung oder andere Methoden der Präzipitation, die die Zielproteine nicht mehrheitlich denaturieren, wie die Fällung mit Hilfe von Ammoniumsulfat oder anderen Verfahren der Aussalzung.

Bei den Präzipitaten der Cohn Fraktionierung handelt es ich um dem Fachmann vertraute Fraktionen: Cryo-Präzipitat, Fraktion I, Fraktion II, Fraktion II+III, Fraktion I+II+III, Fraktion I+III, Fraktion III, Fraktion IV und Fraktion V. Ebenfalls vertraut ist der Fachmann mit den Fraktionen nach Kistler-Nitschmann, wie Cryopräzipitat Präzipitate A, B und C.

Besonders bevorzugt ist die Gewinnung von Immunglobulinen nach dem erfindungsgemäßen Verfahren aus den Fraktionen I+II+III bzw. II+III und schließlich Fraktion II nach Cohn, sowie aus Präzipitat A nach KN. Alternativ oder zusätzlich zu den ausgeführten Fraktionen können immunglobulinhaltige Präzipitate hergestellt durch Polyethylenfällung, Aussalzen oder andere Präzipitationsverfahren Verwendung finden.

Die Erfindung wird an hand des folgenden Beispieles näher erläutert:

### Beispiel1:

Fraktion I+II+III wurde nach dem Fachmann vertrauten Cohn-Verfahren gewonnen und zu Fraktion II nach Cohn weiterverarbeitet, die den Hauptanteil an Immunglobulin vom G (IgG) enthält.

Diese Fraktion II wurde in gleiche Teile geteilt, mit denen in unterschiedlicher Weise verfahren wurde:
1. Einfrieren: ≤ -25° C; Lagerung: ≤ -25° C
2. Einfrieren: Flüssiger Stickstoff; Lagerung: ≤ -70° C

Na Lagerung für drei Monate unter den o.g Bedingungen, wurden die Fraktionen nach dem identischen Verfahren rekonstltuiert. das heißt im Wesentlichen Immunglobuline wurden nach dem Fachmann bekannten Cohn-Verfahren in identischen Volumina gelöst und danach filtriert, um schlecht lösliche Bestandteile zu entfernen.

### Ergebnis:

Die durch Rekonstitution der verschieden gelagerten Präzipitate erhaltenen IgG haltigen Lösungen zeigten bei Analyse hinsichtlich der Produktqualität keine signifikanten Unterschiede. In Ansatz 2 wurde jedoch eine signifikant höhere Ausbeute an IgG erzielt. Die Steigerung der Ausbeute betrug bis zu 20% derjenigen, die bei Ausführung von Ansatz 1 erzielt wurde.

## Patentansprüche

1. Verfahren zur Steigerung der Ausbeute von Proteinen, insbesondere Plasmaproteinen aus die Proteine, insbesondere Plasmaproteine enthaltenden Quellen, wobei die Proteine enthaltenden Quellen bei Temperaturen ≤ -70° C eingefroren werden und die Proteine aus einer aufgetauten eingefrorenen Quelle in an sich bekannter Weise weiter aufgearbeitet werden.

2. Verfahren nach Anspruch 1, wobei die Proteine enthaltenden Quellen mit flüssigem Stickstoff eingefroren werden.

3. Verfahren nach Anspruch 1 und/oder 2, wobei Plasmaproteine ausgewählt sind aus der Gruppe bestehend aus Immunglobulinen aller Klassen und Subtypen, Gerinnungsfaktoren, andere in Gerinnung oder Fibrinolyse involvierte Proteine, Albumin und Wundverschluss oder Wundheilung fördernde Substanzen sowie Proteine mit Transportfunktion.

4. Verfahren nach Anspruch 3, wobei die Immunglobuline ausgewählt sind aus der Gruppe bestehend aus IgG, IgM, IgA, IgE und deren Subtypen.

5. Verfahren nach Anspruch 3, wobei die Gerinnungsfaktoren ausgewählt sind aus der Gruppe bestehend aus den Faktoren II, V, VII, VIII, IX, X, XI, XII, XIII, Fibrinogen und von Willebrand Factor.

6. Verfahren nach Anspruch 3, wobei die anderen in Gerinnung oder Fibrinolyse involvierten Proteine ausgewählt sind aus der Gruppe bestehend aus Plasminogen, Faktor VII aktivierende Protease, Proteaseinhibitoren, wie Alpha-1-Antitrypsin, Antithrombin III oder C 1-Esteraseinhibitor und Alpha-2-Antiplasmin.

7. Verfahren nach Anspruch 3, wobei die den Wundverschluss oder die Wundheilung fördernden Substanzen ausgewählt sind aus der Gruppe bestehend aus Fibronektin, Wachstumsfaktoren wie HGF, FGF oder PDGF.

8. Verfahren nach Anspruch 3, wobei die Proteine mit Transportfunktion ausgewählt sind aus der Gruppe bestehend aus Transferrin, Faktoren des Komplementsystemes oder Histidin-reiches Glykoprotein.

9. Verfahren nach mindestens einem der Ansprüche 1 bis 8, wobei die Plasmaproteine enthaltenden Quellen ausgewählt sind aus der Gruppe bestehend aus Cryopräzipitat, Cohn Fraktionen I, II, III, I+III, II+III, I+II+III, IV, V sowie Kombinationen davon oder aus Kistler-Nitschmann Fraktionen I, IV, Cryopräzipitat, Präzipitaten A, B, C, D, G(G) sowie deren Kombinationen und Modifikationen.

10. Verfahren nach mindestens einem der Ansprüche 1 bis 8, wobei die Plasmaproteine enthaltenden Quellen aus Protein-Präzipitaten hergestellt werden, die die herzustellenden Plasmaproteine weniger als 50% denaturieren.

11. Verfahren nach Anspruch 10, wobei die Protein-Präzipitate durch Zugabe von Polyethylenglykol zu den die Plasmaproteine enthaltenden Quellen und/oder Aussalzen von Proteinen aus den die Plasmaproteine enthaltenden Quellen erhältlich sind.

12. Verfahren nach mindestens einem der Ansprüche 1 bis 11, wobei eine Lagerung der bei Temperaturen ≤ -70° C eingefrorenen Plasmaproteine enthaltenden Quellen bei Temperaturen ≤ -18° C erfolgt, insbesondere bei ≤ -70° C.

13. Verfahren nach mindestens einem der Ansprüche 1 bis 12, wobei Plasmaproteine enthaltende Lösungen, Pasten Intermediaten aus der Cohn-Fraktionierung oder Kistler-Nitschmann Fraktionierung stammenden Fraktionen eingefroren werden.

14. Verfahren nach Anspruch 13, wobei die Lagerung für eine Zeit von mindestens 12 Stunden erfolgt.

15. Verfahren nach mindestens einem der Ansprüche 1 bis 14, wobei proteinhaltige Fraktionen aus rekombinant und transgen hergestellten Präzipitaten und Lösungen eingefroren und gelagert werden.
